# EUROPEAN PATENT APPLICATION

(11) **EP 4 566 528 A1**
(43) Date of publication of application: **11.06.2025**
(21) Application number: 23850209.0
(22) Date of filing: 17.05.2023
(51) Int. Cl.: A61B 5/026, A61B 5/00, A61B 5/055, A61B 6/03, A61B 6/00, G16H 50/20

(54) **METHOD FOR COMPUTING CEREBRAL BLOOD FLOW DATA AND METHOD FOR TRAINING NEURAL NETWORK MODEL FOR COMPUTING CEREBRAL BLOOD FLOW DATA**

(30) Priority: 02.08.2022 KR 20220096054
(71) Applicant: Near Brain Inc., Gangnam-gu Seoul 06029 (KR)
(72) Inventor: LEE, Tae Rin, Seoul 03068 (KR)
(74) Representative: Nederlandsch Octrooibureau
(86) International application number: PCT/KR2023/006676
(87) International publication number: WO 2024/029698

(57) **Abstract**

A method for computing cerebral blood flow data according to an embodiment of the present disclosure includes the steps of: acquiring a trained cerebral blood flow prediction model; acquiring an original medical image; acquiring morphological data corresponding to the cerebral blood vessel region from the original medical image, the morphological data being composed of data in the form of a point cloud; acquiring a boundary area of the cerebral blood vessel region and acquiring boundary information corresponding to the boundary area; acquiring initial condition information or boundary condition information; and inputting the morphological data, the boundary information, and the initial condition information or boundary condition information into the cerebral blood flow prediction model, and acquiring cerebral blood flow data related to the speed or pressure of the cerebral blood flow output through the cerebral blood flow prediction model.

## Description

### Technical Field

The present disclosure relates to a method of computing cerebral blood flow data. Specifically, the present disclosure relates to a method and device for training a neural network model for computing cerebral blood flow data and a method of computing cerebral blood flow data using a neural network model which has been trained.

### Background Art

Various imaging techniques, such as positron emission tomography (PET), functional magnetic resonance imaging (fMRI), magnetic resonance angiography (MRA), and computed tomography (CT), have been developed to visualize three-dimensional (3D) structures of a brain and cerebral blood vessels. These advances in imaging technologies have made it possible to measure cerebral blood flow throughout the brain.

Thus far, cerebral blood flow has been computed using blood flow computation software or any mathematical/statistical technique. However, existing methods have a limitation in that, when new data is acquired, it is necessary to newly compute cerebral blood flow using computation software or a mathematical/statistical technique. In addition, existing cerebral blood flow computation methods have the technical barrier and inconvenience of having to generate a surface model or solid model related to brain shapes to compute cerebral blood flow.

Accordingly, it is necessary to develop a new technology for efficiently computing cerebral blood flow.

### Disclosure

### Technical Problem

The present disclosure is directed to providing a method of computing cerebral blood flow and a method of training a neural network model for computing cerebral blood flow data.

Objects to be achieved by the present disclosure are not limited to that described above, and other objects which have not been described will be clearly understood by those skilled in the technical field to which the present disclosure pertains from the present specification and the accompanying drawings.

### Technical Solution

This summary is provided to introduce a selection of concepts in a simplified form that are further explained in the detailed description below. This summary is not intended to identify the main features or essential features of the claimed subject matter and is not intended to assist in determining the scope of the claimed subject matter.

One aspect of the present disclosure provides a method of computing cerebral blood flow data, the method including acquiring a cerebral blood flow prediction model which has been trained, acquiring an original medical image, acquiring morphological data, which is composed of data in the form of a point cloud, corresponding to a cerebrovascular region from the original medical image, acquiring a boundary region of the cerebrovascular region and acquiring boundary information corresponding to the boundary region, acquiring initial condition information or boundary condition information, and inputting the morphological data, the boundary information, and the initial condition information or the boundary condition information into the cerebral blood flow prediction model and acquiring cerebral blood flow data related to a cerebral blood flow speed or a cerebral blood flow pressure output from the cerebral blood flow prediction model.

Another aspect of the present disclosure provides a method of computing cerebral blood flow data, the method including acquiring a cerebral blood flow prediction model which has been trained, acquiring an original medical image, acquiring first morphological data, which is composed of node information and connectivity information constituting a one-dimensional (1D) network, corresponding to a cerebrovascular region from the original medical image, acquiring a boundary region of the cerebrovascular region and acquiring boundary information corresponding to the boundary region, acquiring initial condition information or boundary condition information, and inputting the first morphological data, the boundary information, and the initial condition information or the boundary condition information into the cerebral blood flow prediction model and acquiring cerebral blood flow data related to a cerebral blood flow speed or a cerebral blood flow pressure output from the cerebral blood flow prediction model.

Technical solutions of the present disclosure are not limited to those described above, and other technical solutions which have not been described will be clearly understood by those skilled in the technical field to which the present disclosure pertains from the present specification and the accompanying drawings.

### Advantageous Effects

With a method of computing cerebral blood flow data, a method of training a cerebral blood flow prediction model, and a cerebral blood flow data computation device according to an embodiment of the present disclosure, it is possible to train a cerebral blood flow prediction model capable of computing cerebral blood flow information from a point model or a one-dimensional (1D) network model rather than a surface model or a solid model.

With a method of computing cerebral blood flow data, a method of training a cerebral blood flow prediction model, and a cerebral blood flow data computation device according to an embodiment of the present disclosure, it is possible to automatically compute cerebral blood flow information from a point model or a 1D network model rather than a surface model or a solid model using a cerebral blood flow prediction model which has been trained.

Effects of the present disclosure are not limited to those described above, and other effects which have not been described will be clearly understood by those skilled in the technical field to which the present disclosure pertains from the present specification and the accompanying drawings.

### Description of Drawings

FIG. 1 is a schematic diagram of a cerebral blood flow computation device according to an embodiment of the present disclosure.
FIG. 2 is a schematic diagram illustrating an operation of the cerebral blood flow computation device according to the embodiment of the present disclosure.
FIG. 3 is a diagram illustrating an aspect of computing cerebral blood flow data using a first cerebral blood flow prediction model according to the embodiment of the present disclosure.
FIG. 4 is a diagram illustrating an aspect of training a first cerebral blood flow prediction model according to the embodiment of the present disclosure.
FIG. 5 is a diagram illustrating another aspect of training a first cerebral blood flow prediction model according to the embodiment of the present disclosure.
FIG. 6 is a diagram illustrating an aspect of training a first cerebral blood flow prediction model and an aspect of validating the performance of the first cerebral blood flow prediction model according to the embodiment of the present disclosure.
FIG. 7 is a diagram illustrating an aspect of computing cerebral blood flow data using a second cerebral blood flow prediction model according to another embodiment of the present disclosure.
FIG. 8 is a diagram illustrating an aspect of training a second cerebral blood flow prediction model according to the other embodiment of the present disclosure.
FIG. 9 is a diagram illustrating another aspect of training a second cerebral blood flow prediction model according to the other embodiment of the present disclosure.
FIG. 10 is a diagram illustrating an aspect of training a second cerebral blood flow prediction model and an aspect of validating the performance of the second cerebral blood flow prediction model according to the other embodiment of the present disclosure.
FIG. 11 is a flowchart illustrating a method of computing cerebral blood flow data using a first cerebral blood flow prediction model according to the embodiment of the present disclosure.
FIG. 12 is a flowchart illustrating a method of computing cerebral blood flow data using a second cerebral blood flow prediction model according to the other embodiment of the present disclosure.

### mode for Invention

The above-described technical objects, features, and advantages of the present disclosure will be more obvious from the following detailed description in conjunction with the accompanying drawings. However, the present disclosure can be modified in various ways and have several embodiments, and specific embodiments will be illustrated in the drawings and described in detail below.

Throughout the specification, like reference numerals refer to like components in principle. Also, components having the same function within the scope of the same idea illustrated in the drawings of embodiments will be described using the same reference numeral, and reiterative description thereof will be omitted.

When detailed description of a known function or element related to the present disclosure is determined to unnecessarily obscure the subject matter of the present disclosure, the detailed description will be omitted. Also, numbers (e.g., first, second, and the like) used in the description process of the present specification are merely identification code for distinguishing one component from others.

In addition, the suffixes "module" and "unit" for components used in the following embodiments are given or used together only to facilitate the writing of the specification. Therefore, these terms do not have distinct meanings or roles therein.

In the following embodiments, singular forms include plural forms unless the context clearly indicates otherwise.

In the following embodiments, the term "include," "have" or the like means that a feature or component described in the specification is present, and it does not preclude the possibility that one or more other features or components will be added.

For convenience of description, the sizes of components may be exaggerated or reduced in the accompanying drawings. For example, the size and thickness of each component illustrated in the drawings are arbitrarily indicated for convenience of description, and the present disclosure is not necessarily limited to what is illustrated.

When an embodiment can be implemented differently, a specific process may be performed in a different order from the described order of the process. For example, two processes described in succession may be performed substantially simultaneously, or may be performed in a reverse order to the described order.

In the following embodiments, when components and the like are referred to as being connected, the components may not only be directly connected but may also be indirectly connected with components interposed therebetween.

For example, in the present specification, when components and the like are referred to as being electrically connected, the components may not only be directly electrically connected but may also be indirectly electrically connected with components interposed therebetween.

A method of computing cerebral blood flow data according to an embodiment of the present disclosure may include an operation of acquiring a cerebral blood flow prediction model which has been trained, an operation of acquiring an original medical image, an operation of acquiring morphological data, which is composed of data in the form of a point cloud, corresponding to a cerebrovascular region from the original medical image, an operation of acquiring a boundary region of the cerebrovascular region and acquiring boundary information corresponding to the boundary region, an operation of acquiring initial condition information or boundary condition information, and an operation of inputting the morphological data, the boundary information, and the initial condition information or the boundary condition information into the cerebral blood flow prediction model and acquiring cerebral blood flow data related to a cerebral blood flow speed or a cerebral blood flow pressure output from the cerebral blood flow prediction model.

According to an embodiment of the present disclosure, the cerebral blood flow prediction model may be trained on the basis of a training dataset including first data related to morphological data in the form of a point cloud corresponding to a cerebrovascular region, second data related to boundary information of the cerebrovascular region, third data related to initial condition information or boundary condition information, and fourth data related to a cerebral blood flow prediction value regarding a cerebral blood flow speed or pressure.

According to an embodiment of the present disclosure, the cerebral blood flow prediction model may be configured to receive the training dataset and output an output value from the training dataset, and parameters included in the cerebral blood flow prediction model may be updated to output the output value approximating the cerebral blood flow prediction value such that the cerebral blood flow prediction model may be trained.

According to an embodiment of the present disclosure, the fourth data may be generated by interpolating a blood flow speed or a blood flow pressure computed from the morphological data corresponding to the cerebrovascular region to the morphological data having the form of a point cloud, and the computed blood flow speed or blood flow pressure may be computed on the basis of a surface model generated from the morphological data using computational fluid dynamics (CFD).

According to an embodiment of the present disclosure, the fourth data may be generated by interpolating a blood flow speed or a blood flow pressure computed from the morphological data corresponding to the cerebrovascular region to the morphological data having the form of a point cloud, and the computed blood flow speed or blood flow pressure may be computed on the basis of a one-dimensional (1D) network model generated from the morphological data.

A method of computing cerebral blood flow data according to another embodiment of the present disclosure may include an operation of acquiring a cerebral blood flow prediction model which has been trained, an operation of acquiring an original medical image, an operation of acquiring first morphological data, which is composed of node information and connectivity information constituting a 1D network model, corresponding to a cerebrovascular region from the original medical image, an operation of acquiring a boundary region of the cerebrovascular region and acquiring boundary information corresponding to the boundary region, an operation of acquiring initial condition information or boundary condition information, and an operation of inputting the first morphological data, the boundary information, and the initial condition information or the boundary condition information into the cerebral blood flow prediction model and acquiring cerebral blood flow data related to a cerebral blood flow speed or a cerebral blood flow pressure output from the cerebral blood flow prediction model.

According to the other embodiment of the present disclosure, the cerebral blood flow prediction model may be trained on the basis of a training dataset including first data composed of node information and connectivity information constituting a 1D network model, second data related to boundary information of the cerebrovascular region, third data related to initial condition information or boundary condition information, and fourth data related to a cerebral blood flow prediction value regarding a cerebral blood flow speed or pressure.

According to the other embodiment of the present disclosure, the cerebral blood flow prediction model may be configured to receive the training dataset and output an output value from the training dataset, and parameters included in the cerebral blood flow prediction model may be updated to output the output value approximating the cerebral blood flow prediction value such that the cerebral blood flow prediction model may be trained.

According to the other embodiment of the present disclosure, the fourth data may be generated by interpolating a blood flow speed or a blood flow pressure computed from second morphological data having the form of a point cloud corresponding to the cerebrovascular region to the first morphological data having the form of the 1D network model, and the computed blood flow speed or blood flow pressure may be computed on the basis of a surface model generated from the second morphological data using CFD.

According to the other embodiment of the present disclosure, the fourth data may be generated by computing a cerebral blood flow prediction value related to a blood flow speed or a blood flow pressure from the first morphological data.

According to an embodiment of the present disclosure, a computer-readable recording medium on which a program for executing the method of computing cerebral blood flow data is recorded may be provided.

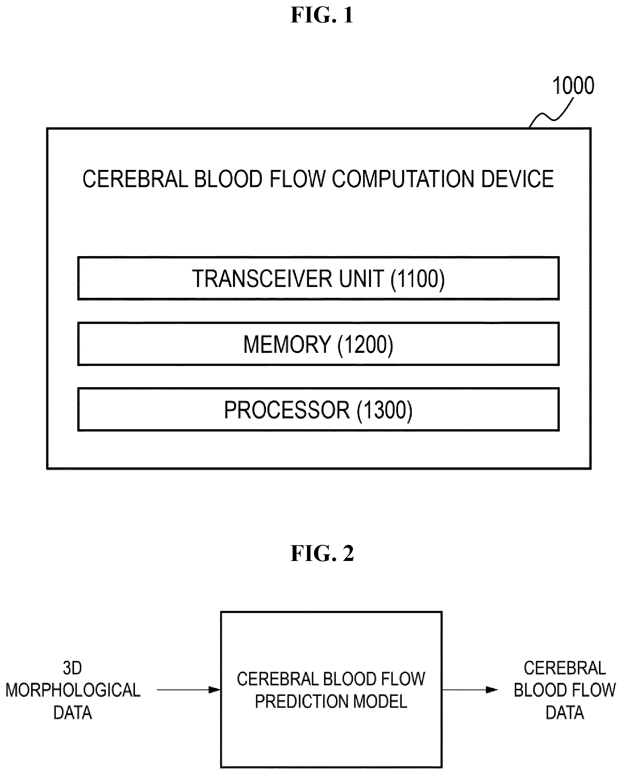

Hereinafter, a method of computing cerebral blood flow data, a method of training a cerebral blood flow prediction model, and a cerebral blood flow computation device (or cerebral blood flow computation server, hereinafter, "cerebral blood flow computation device") according to embodiments of the present disclosure will be described with reference to FIGS. 1 to 12.

FIG. 1 is a schematic diagram of a cerebral blood flow computation device according to an embodiment of the present disclosure.

A cerebral blood flow computation device 1000 according to an embodiment of the present disclosure may train a cerebral blood flow prediction model. Also, the cerebral blood flow computation device 1000 may compute or predict cerebral blood flow data from an original medical image using the cerebral blood flow prediction model which has been trained.

The cerebral blood flow computation device 1000 according to an embodiment of the present disclosure may include a transceiver unit 1100, a memory 1200, and a processor 1300.

The transceiver unit 1100 may communicate with any external device including medical imaging equipment (e.g., magnetic resonance angiography (MRA) equipment, magnetic resonance imaging (MRI) equipment, computed tomography (CT) equipment, positron emission tomography (PET) equipment, and the like). For example, the cerebral blood flow computation device 1000 may acquire a medical image captured by the medical imaging equipment through the transceiver unit 1100. Also, the cerebral blood flow computation device 1000 may acquire any execution data for executing the trained cerebral blood flow prediction model through the transceiver unit 1100. Here, the execution data may be any suitable data for executing the cerebral blood flow prediction model, including structural information, hierarchy information, and computational libraries of the cerebral blood flow prediction model and a set of parameters related to weights included in the cerebral blood flow prediction model. Also, the cerebral blood flow computation device 1000 may transmit or output cerebral blood flow data acquired through the cerebral blood flow prediction model to any external device including a user terminal through the transceiver unit 1100.

The cerebral blood flow computation device 1000 may access a network through the transceiver unit 1100 to transmit and receive various data. In general, the transceiver unit 1100 may be a wired type transceiver unit or a wireless type transceiver unit. Since the wired-type transceiver unit and the wireless-type transceiver unit each have advantages and disadvantages, the cerebral blood flow computation device 1000 may be provided with both the wired-type transceiver unit and the wireless-type transceiver unit in some cases. Here, the wireless-type transceiver unit may mainly use a wireless local area network (WLAN)-based communication scheme such as Wi-Fi. Alternatively, the wireless-type transceiver unit may use a cellular communication scheme, for example, a Long-Term Evolution (LTE) or fifth generation (5G) communication scheme. However, a wireless communication protocol is not limited to the above examples, and it is possible to use any appropriate wireless-type communication scheme. Representative examples of the wired-type transceiver unit employ local area network (LAN) and Universal Serial Bus (USB) communication, and other communication schemes are also available.

The memory 1200 may store various information. Various data may be temporarily or semi-temporarily stored in the memory 1200. Examples of the memory 1200 may be a hard disk drive (HDD), a solid state drive (SSD), a flash memory, a read-only memory (ROM), a random access memory (RAM), and the like. The memory 1200 may be provided in a form that is embedded into or detachable from the cerebral blood flow computation device 1000. Various data necessary for operations of the cerebral blood flow computation device 1000, such as an operating system (OS) for operating the cerebral blood flow computation device 1000 and a program for operating each element of the cerebral blood flow computation device 1000, may be stored in the memory 1200.

The processor 1300 may control overall operations of the cerebral blood flow computation device 1000. For example, the processor 1300 may control overall operations of the cerebral blood flow computation device 1000, which will be described below, and the operations include an operation of acquiring three-dimensional (3D) morphological data from an original medical image, an operation of acquiring boundary information, an operation of acquiring an initial condition or a boundary condition, an operation of training a cerebral blood flow prediction model, and an operation of computing cerebral blood flow data through the cerebral blood flow prediction model which has been trained. Specifically, the processor 1300 may load a program for the overall operations of the cerebral blood flow computation device 1000 from the memory 1200 and execute the program. The processor 1300 may be implemented as an application processor (AP), a central processing unit (CPU), or a similar device in accordance with hardware, software, or a combination thereof. As hardware, the processor 1300 may be provided in the form of an electronic circuit for processing an electrical signal to perform a control function, and as software, may be provided in the form of a program or code for operating a hardware circuit.

FIG. 2 is a schematic diagram illustrating an operation of a cerebral blood flow computation device according to the embodiment of the present disclosure.

The cerebral blood flow computation device 1000 according to the embodiment of the present disclosure may compute cerebral blood flow data from 3D morphological data using a cerebral blood flow prediction model which has been trained. Specifically, the cerebral blood flow computation device 1000 may detect a region corresponding to cerebral blood vessels from pixel information of an original medical image and acquire morphological data of the detected cerebrovascular region. Here, the pixel information may include coordinate information composed of x-coordinates, y-coordinates, and z-coordinates in the original medical image and gray level information of the original medical image. Also, the cerebral blood flow computation device 1000 may be configured to input 3D morphological data corresponding to the cerebrovascular region into the cerebral blood flow prediction model which has been trained. Here, the cerebral blood flow computation device 1000 may acquire cerebral blood flow data output from the cerebral blood flow prediction model.

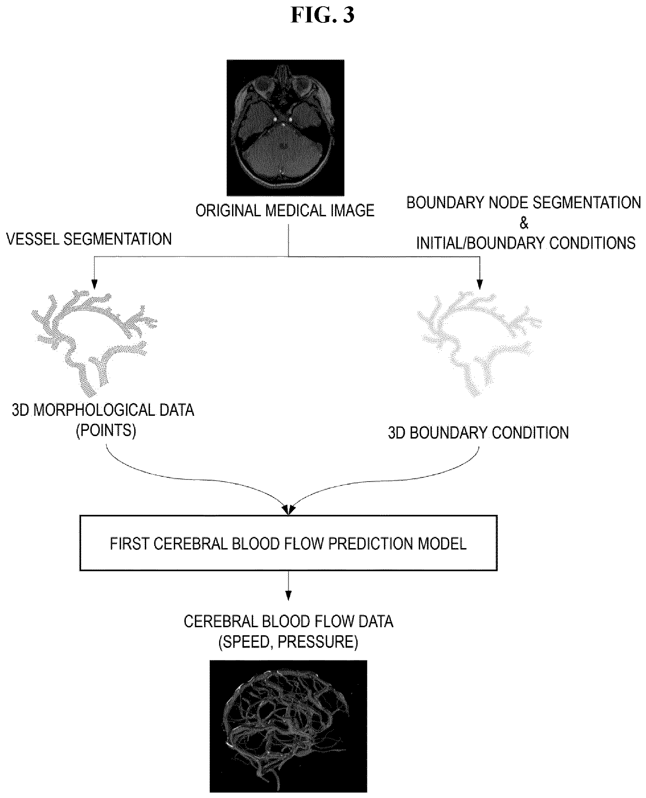

According to an embodiment, the cerebral blood flow computation device 1000 may compute cerebral blood flow data on the basis of 3D morphological data composed in the form of a point cloud. This will be described in further detail below with reference to FIGS. 3 to 6.

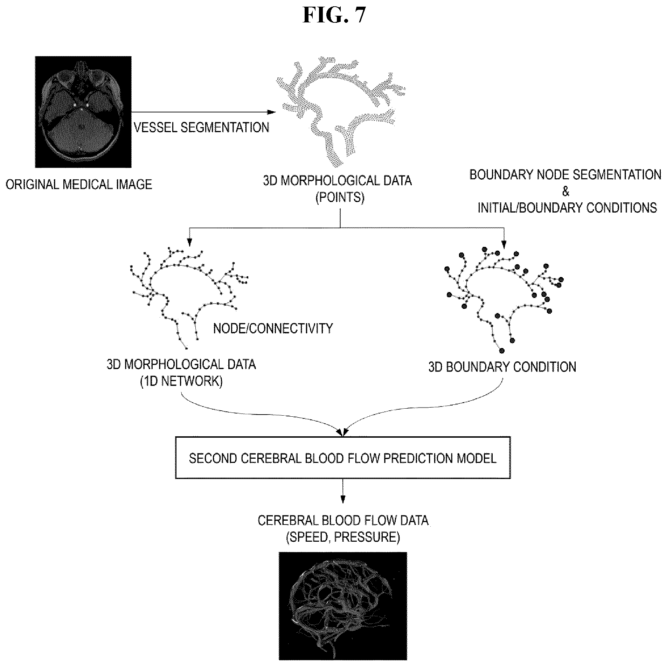

According to another embodiment, the cerebral blood flow computation device 1000 may compute cerebral blood flow data on the basis of 3D morphological data composed in the form of a 1D network in which points (or nodes) included in coordinate information corresponding to the cerebrovascular region are connected. This will be described in further detail below with reference to FIGS. 7 to 10.

Various operations of the cerebral blood flow computation device 1000, a method of computing cerebral blood flow data, and a method of training a cerebral blood flow prediction model according to the embodiment of the present disclosure will be described in detail below with reference to FIGS. 3 to 6. FIG. 3 is a diagram illustrating an aspect of computing cerebral blood flow data using a first cerebral blood flow prediction model according to the embodiment of the present disclosure. According to the present embodiment, it is possible to compute cerebral blood flow data from 3D morphological data in the form of a point cloud through the first cerebral blood flow prediction model which has been trained.

The cerebral blood flow computation device 1000 according to the embodiment of the present disclosure may acquire an original medical image and acquire a region of interest (e.g., a region corresponding to cerebral blood vessels) from pixel information of the original medical image. For example, the cerebral blood flow computation device 1000 may acquire a region of interest using an image segmentation technique and acquire coordinate information corresponding to the region of interest. As an example, the cerebral blood flow computation device 1000 may acquire coordinate information of the region of interest (e.g., a region corresponding to cerebral blood vessels) from pixel information of the medical image in which gray level information has a value equal to or less than a predetermined value. As described above, the coordinate information may be x-coordinates, y-coordinates, and z-coordinates of pixels constituting the original medical image. Further, the cerebral blood flow computation device 1000 may acquire 3D morphological data (e.g., also referred to as point information or a point model) in the form of a point cloud on the basis of the coordinate information of the region of interest.

The cerebral blood flow computation device 1000 according to the embodiment of the present disclosure may acquire a boundary region of the region of interest (e.g., a region corresponding to cerebral blood vessels) and acquire boundary information (e.g., coordinate information of the boundary region) corresponding to the boundary region. Specifically, the cerebral blood flow computation device 1000 may acquire nodes corresponding to the boundary region of the region of interest (e.g., a cerebrovascular region) using an image segmentation technique and acquire the boundary information. Further, the cerebral blood flow computation device 1000 according to the embodiment of the present disclosure may acquire a 3D initial condition and/or boundary condition related to the boundary region.

The cerebral blood flow computation device 1000 according to the embodiment of the present disclosure may compute cerebral blood flow data using the first cerebral blood flow prediction model which has been trained. Specifically, the cerebral blood flow computation device 1000 may input the 3D morphological data (e.g., a points model), the boundary information, and/or the 3D initial condition and boundary condition information into the first cerebral blood flow prediction model which has been trained, and acquire cerebral blood flow data related to a cerebral blood flow speed or a cerebral blood flow pressure output from the first cerebral blood flow prediction model which has been trained. Since the first cerebral blood flow prediction model is trained to output a cerebral blood flow prediction value related to a cerebral blood flow speed and pressure on the basis of points information, boundary information, and/or 3D initial condition and boundary condition information, which will be described below, the trained first cerebral blood flow prediction model can output cerebral blood flow data related to a cerebral blood flow speed and pressure from 3D morphological data in the form of a point cloud corresponding to a cerebrovascular region, boundary information of the cerebrovascular region, and/or initial condition or boundary condition information.

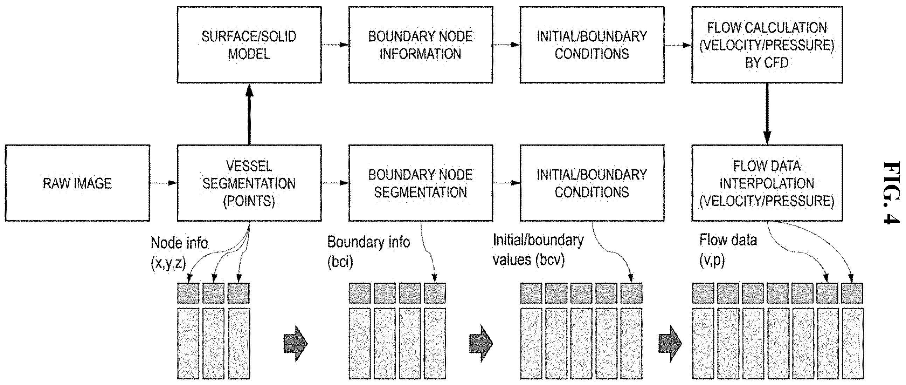

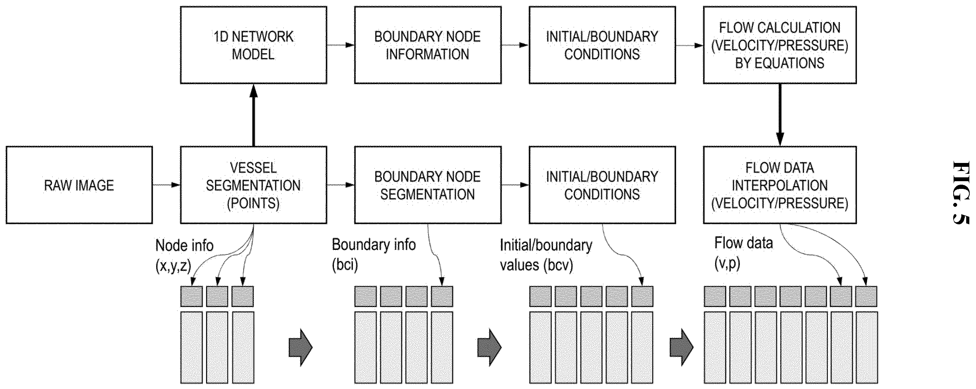

A method of training a first cerebral blood flow prediction model and a method of generating a training dataset according to the embodiment of the present disclosure will be described below with reference to FIGS. 4 and 5. According to the embodiment of the present disclosure, a training dataset for training a first cerebral blood flow prediction model includes data related to a cerebral blood flow prediction value.

According to an aspect, a cerebral blood flow prediction value may be calculated using a surface model and/or a solid model. This will be described in further detail with reference to FIG. 4.

According to another aspect, a cerebral blood flow prediction value may be calculated using a 1D network model. This will be described in further detail with reference to FIG. 5.

FIG. 4 is a diagram illustrating an aspect of training a first cerebral blood flow prediction model according to the embodiment of the present disclosure.

A first cerebral blood flow prediction model may be trained on the basis of a first training dataset that includes first data (Node info (x, y, z) of FIG. 4) related to 3D morphological data in the form of a point cloud corresponding to a cerebrovascular region, second data (Boundary info of FIG. 4) related to boundary information of the cerebrovascular region, third data related to initial condition and boundary condition information (Initial/boundary values (bcv) of FIG. 4), and fourth data (Flow data (v, p) of FIG. 4) related to a blood flow speed and pressure. Specifically, the cerebral blood flow computation device 1000 may generate the first data, the second data, and the third data from the original medical image (raw image) as described above with reference to FIG. 3.

Further, the cerebral blood flow computation device 1000 may calculate a cerebral blood flow prediction value using a surface model and/or a solid model and acquire fourth data (Flow data (v, p) of FIG. 4) related to a blood flow speed and pressure by interpolating the cerebral blood flow prediction value to the 3D morphological data having the form of a point cloud. Specifically, the cerebral blood flow computation device 1000 may generate a surface model or a solid model that presents a cerebrovascular morphology from coordinate information corresponding to a region of interest using a computer vision segmentation technique. For example, the cerebral blood flow computation device 1000 may generate a surface model for the region of interest on the basis of coordinates constituting one surface in coordinate information. Meanwhile, the original medical image may be composed of a set of 2D images, thus including 3D coordinate information. Here, the cerebral blood flow computation device 1000 may generate a 3D surface model that presents the morphology of the region of interest (e.g., a cerebrovascular region) on the basis of 3D coordinate information.

Also, the cerebral blood flow computation device 1000 may acquire a boundary region of the region of interest (e.g., a cerebrovascular region) from the surface model (or solid model) and acquire boundary information (e.g., coordinate information or node information of the boundary region) corresponding to the boundary region. Specifically, the cerebral blood flow computation device 1000 may acquire nodes corresponding to the boundary region of the region of interest (e.g., a cerebrovascular region) using any segmentation technique and acquire boundary information. Further, the cerebral blood flow computation device 1000 according to the embodiment of the present disclosure may acquire a 3D initial condition and/or boundary condition related to the boundary region.

In addition, the cerebral blood flow computation device 1000 may compute a cerebral blood flow prediction value regarding a cerebral blood flow speed or pressure from the surface model (or solid model) using computational fluid dynamics (CFD) or computational aided engineering (CAE). Here, the cerebral blood flow computation device 1000 may interpolate the computed cerebral blood flow prediction value to 3D morphological data having the form of a point cloud to generate fourth data (Flow data (v, p) of FIG. 4) related to a blood flow speed and pressure.

Further, the cerebral blood flow computation device 1000 may input a first training dataset that includes the first data related to the 3D morphological data (Node info (x, y, z) of FIG. 4) having the form of a point cloud, the second data related to boundary information (bci of FIG. 4), the third data related to the initial condition and boundary condition (bcv of FIG. 4), and the fourth data ((v, p) of FIG. 4) related to a cerebral blood flow prediction value, into the first cerebral blood flow prediction model and acquire an output value output from the first cerebral blood flow prediction model. Here, the cerebral blood flow computation device 1000 may compare the output value with the cerebral blood flow prediction value included in the fourth data and update parameters included in the first cerebral blood flow prediction model such that the first cerebral blood flow prediction model may output an output value approximating the cerebral blood flow prediction value.

FIG. 5 is a diagram illustrating another aspect of training a first cerebral blood flow prediction model according to the embodiment of the present disclosure.

Similarly to the description provided with reference to FIG. 4, the first cerebral blood flow prediction model may be trained on the basis of a first training dataset including first data (Node info (x, y, z) of FIG. 5) related to 3D morphological data in the form of a point cloud corresponding to a cerebrovascular region, second data (Boundary info (bci) of FIG. 5) related to boundary information of the cerebrovascular region, third data related to initial condition and boundary condition information (Initial/boundary values (bcv) of FIG. 5), and fourth data (Flow data (v, p) of FIG. 5) related to a blood flow speed and pressure.

Meanwhile, according to an aspect, the cerebral blood flow computation device 1000 may calculate a cerebral blood flow prediction value using a 1D network model and interpolate the cerebral blood flow prediction value to the 3D morphological data having the form of a point cloud to acquire the fourth data (Flow data (v, p) of FIG. 5) related to a blood flow speed and pressure. Specifically, the cerebral blood flow computation device 1000 may generate a 1D network model that presents a cerebrovascular morphology from coordinate information corresponding to a region of interest (e.g., a cerebrovascular region). Here, the 1D network model may be a model in which points corresponding to coordinate information of a segmented region of interest are connected using any method, and the 1D network model may be composed of node information and connectivity information that presents connection between nodes.

Also, the cerebral blood flow computation device 1000 may acquire a boundary region of the region of interest (e.g., a cerebrovascular region) and acquire boundary information (e.g., coordinate information of the boundary region) corresponding to the boundary region. Specifically, the cerebral blood flow computation device 1000 may acquire nodes corresponding to the boundary region of the region of interest (e.g., a cerebrovascular region) using a segmentation technique and acquire boundary information. Further, the cerebral blood flow computation device 1000 according to the embodiment of the present disclosure may acquire a 3D initial condition and/or boundary condition related to the boundary region.

In addition, the cerebral blood flow computation device 1000 may compute a cerebral blood flow prediction value regarding a cerebral blood flow speed or pressure from the 1D network model using any appropriate mathematical technique. For example, the cerebral blood flow computation device 1000 may be configured to assume cerebral blood flow to be Poiseuille flow and compute a cerebral blood flow prediction value regarding a cerebral blood flow speed or pressure using a governing equation. Here, the cerebral blood flow computation device 1000 may interpolate the computed cerebral blood flow prediction value to the 3D morphological data having the form of a point cloud to generate the fourth data (Flow data (v, p) of FIG. 5) related to a blood flow speed and pressure.

Further, similarly to FIG. 4, the cerebral blood flow computation device 1000 may input the first training dataset that includes the first data related to 3D morphological data in the form of a point cloud, the second data related to boundary information, the third data related to an initial condition and boundary condition, and the fourth data related to a cerebral blood flow prediction value, into the first cerebral blood flow prediction model and acquire an output value output from the first cerebral blood flow prediction model. Here, the cerebral blood flow computation device 1000 may compare the output value with the cerebral blood flow prediction value included in the fourth data and update parameters included in the first cerebral blood flow prediction model such that the first cerebral blood flow prediction model may output an output value approximating the cerebral blood flow prediction value.

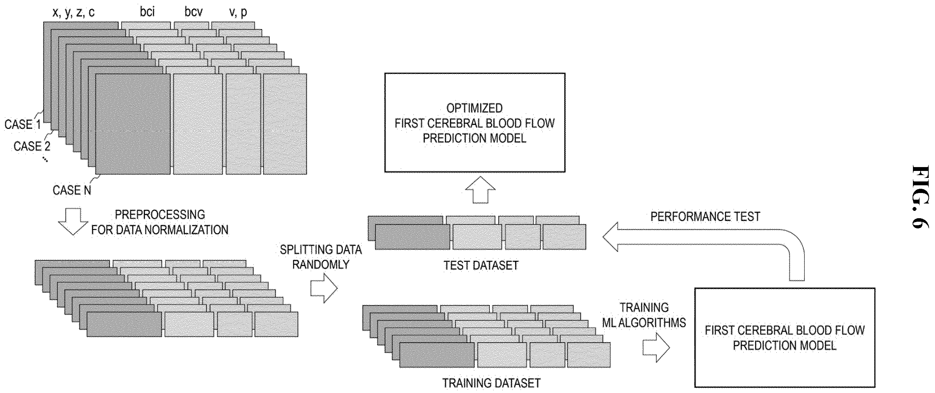

FIG. 6 is a diagram illustrating an aspect of training a first cerebral blood flow prediction model and an aspect of validating the performance of the first cerebral blood flow prediction model according to the embodiment of the present disclosure.

The cerebral blood flow computation device 1000 according to the embodiment of the present disclosure may perform an operation of preprocessing first data related to the 3D morphological data (e.g., Node info (x, y, z)), second data related to boundary information (bci) of a region of interest, third data related to an initial condition or boundary condition (bcv), and/or fourth data related to a cerebral blood flow prediction value (v, p), and the first to fourth data included in a first training dataset. For example, the cerebral blood flow computation device 1000 may normalize the first data, the second data, the third data, and/or the fourth data included in the first training dataset.

Further, the cerebral blood flow computation device 1000 according to the embodiment of the present disclosure may perform an operation of splitting the preprocessed first training dataset into a training dataset and a test dataset or validation dataset. For example, the cerebral blood flow computation device 1000 may randomly split the preprocessed first training dataset into a training dataset and a test dataset. Here, the cerebral blood flow computation device 1000 may train the first cerebral blood flow prediction model through any machine learning (ML) algorithm using the training dataset. Further, the cerebral blood flow computation device 1000 may test or validate the performance of the trained first cerebral blood flow prediction model using the validation dataset. For example, the cerebral blood flow computation device 1000 may compute the performance of the trained first cerebral blood flow prediction model, compare the computed performance with predetermined target performance, and train the first cerebral blood flow prediction model as described above until the computed performance becomes higher than the predetermined target performance, thereby acquiring an optimized first cerebral blood flow prediction model.

Various operations of the cerebral blood flow computation device 1000, a method of computing cerebral blood flow data, and a method of training a cerebral blood flow prediction model according to the other embodiment of the present disclosure will be described in detail below with reference to FIGS. 7 to 10. FIG. 7 is a diagram illustrating an aspect of computing cerebral blood flow data using a second cerebral blood flow prediction model according to the other embodiment of the present disclosure. According to the present embodiment, it is possible to compute cerebral blood flow data from a 1D network model through the second cerebral blood flow prediction model which has been trained.

The cerebral blood flow computation device 1000 according to the other embodiment of the present disclosure may acquire 3D morphological data having the form of a 1D network model from an original medical image. For example, the cerebral blood flow computation device 1000 may acquire a region of interest (e.g., a cerebrovascular region) from the original medical image using an image segmentation technique and acquire coordinate information corresponding to the region of interest having the form of a point cloud. Here, the cerebral blood flow computation device 1000 may connect points included in the coordinate information corresponding to the cerebrovascular region to generate a 1D network model composed of node information corresponding to the region of interest and connectivity information that presents connection between nodes. From the generated 1D network model, the cerebral blood flow computation device 1000 may acquire the node information corresponding to the region of interest (e.g., a cerebrovascular region) and the connectivity information presenting connection between nodes.

Further, as described above with reference to FIG. 3, the cerebral blood flow computation device 1000 according to the other embodiment of the present disclosure may acquire a boundary region of the region of interest and acquire boundary information corresponding to the boundary region. Also, the cerebral blood flow computation device 1000 may acquire a 3D initial condition and/or boundary condition related to the boundary region.

The cerebral blood flow computation device 1000 according to the other embodiment of the present disclosure may compute cerebral blood flow data using the second cerebral blood flow prediction model which has been trained. Specifically, the cerebral blood flow computation device 1000 may input the 3D morphological data having the form of the 1D network model, the boundary information, and/or the 3D initial condition and boundary condition information to the second cerebral blood flow prediction model which has been trained, and acquire cerebral blood flow data related to a cerebral blood flow speed or pressure output from the second cerebral blood flow prediction model which has been trained. Since the second cerebral blood flow prediction model is trained on the basis of the node information and the connectivity information which constitute the 1D network model, the boundary information, and/or the 3D initial condition and boundary condition information to output a cerebral blood flow prediction value related to a cerebral blood flow speed and pressure, which will be described below, the trained second cerebral blood flow prediction model can output cerebral blood flow data related to a cerebral blood flow speed and pressure from the 3D morphological data of the cerebrovascular region related to the node information and the connectivity information constituting the 1D network model, the boundary information of the cerebrovascular region, and/or the initial condition or boundary condition information.

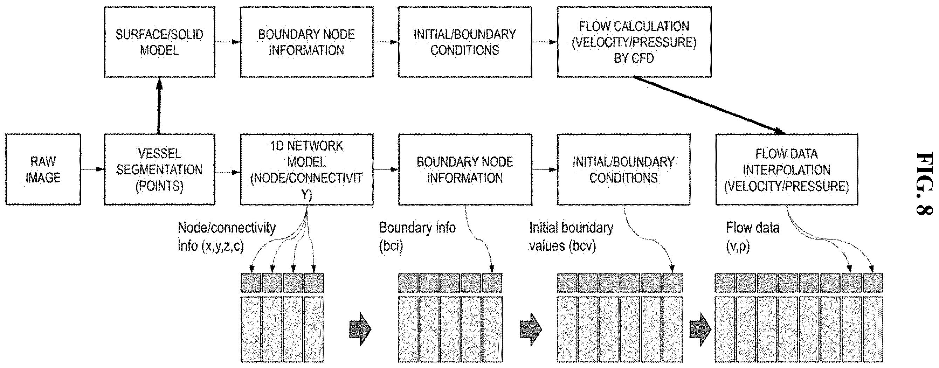

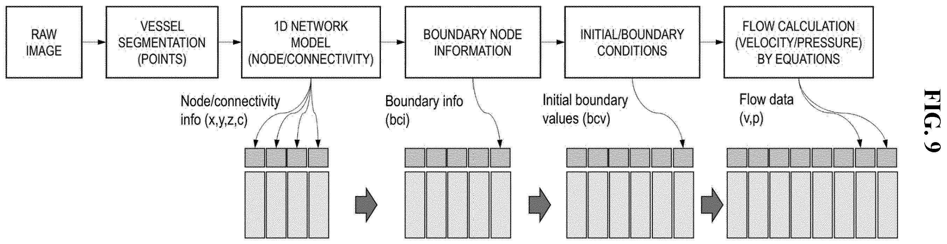

A method of training a second cerebral blood flow prediction model and a method of generating a training dataset according to the other embodiment of the present disclosure will be described below with reference to FIGS. 8 and 9. According to the present embodiment, a training dataset for training the second cerebral blood flow prediction model includes data related to a cerebral blood flow prediction value.

According to an aspect, a cerebral blood flow prediction value may be calculated using a surface model and/or a solid model. This will be described in further detail with reference to FIG. 8.

According to another aspect, a cerebral blood flow prediction value may be calculated using a 1D network model. This will be described in further detail with reference to FIG. 9

FIG. 8 is a diagram illustrating an aspect of training a second cerebral blood flow prediction model according to the other embodiment of the present disclosure.

A second cerebral blood flow prediction model may be trained on the basis of a second training dataset that includes first data (Node/connectivity info (x, y, z, c) of FIG. 8) composed of node information and connectivity information constituting a 1D network model as 3D morphological data having the form of the 1D network model for a cerebrovascular region, second data (Boundary info (bci) of FIG. 8) related to boundary information of the cerebrovascular region, third data related to initial condition and boundary condition information (Initial/boundary values (bcv) of FIG. 8), and fourth data (Flow data (v, p) of FIG. 8) related to a blood flow speed and pressure. Specifically, the cerebral blood flow computation device 1000 may acquire node information and connectivity information of a 1D network model from an original medical image (raw image) to generate first data, acquire boundary information of a cerebrovascular region to generate second data, and acquire a 3D initial condition and boundary condition to generate third data.

Further, as described above with reference to FIG. 4, the cerebral blood flow computation device 1000 may calculate a cerebral blood flow prediction value using a surface model and/or a solid model and acquire fourth data (Flow data (v, p) of FIG. 8) related to a blood flow speed and pressure by interpolating the cerebral blood flow prediction value to the 3D morphological data having the form of the 1D network model. Specifically, the cerebral blood flow computation device 1000 may generate a surface model or a solid model that presents a cerebrovascular morphology from coordinate information corresponding to a region of interest (e.g., a cerebrovascular region) using a computer vision segmentation technique. Also, the cerebral blood flow computation device 1000 may acquire a boundary region of the region of interest (e.g., a cerebrovascular region) from the surface model (or solid model) and acquire boundary information (e.g., coordinate information of the boundary region) corresponding to the boundary region. Specifically, the cerebral blood flow computation device 1000 may acquire nodes corresponding to the boundary region of the region of interest (e.g., a cerebrovascular region) using an image segmentation technique and acquire the boundary information. Further, the cerebral blood flow computation device 1000 may acquire a 3D initial condition and/or boundary condition related to the boundary region. In addition, the cerebral blood flow computation device 1000 may compute a cerebral blood flow prediction value regarding a cerebral blood flow speed or pressure from the surface model (or solid model) using CFD or CAE. Here, the cerebral blood flow computation device 1000 may interpolate the computed cerebral blood flow prediction value to 3D morphological data having the form of a 1D network model to generate fourth data (Flow data (v, p) of FIG. 8) related to a blood flow speed and pressure.

Here, the cerebral blood flow computation device 1000 may input the second training dataset including the first data composed of the node information and the connectivity information constituting the 1D network model, the second data related to the boundary information, the third data related to the initial condition and the boundary condition, and the fourth data related to a blood flow prediction value into the second cerebral blood flow prediction model and acquire an output value output from the second cerebral blood flow prediction model. Here, the cerebral blood flow computation device 1000 may compare the output value with the cerebral blood flow prediction value included in the fourth data and update parameters included in the second cerebral blood flow prediction model such that the second cerebral blood flow prediction model may output an output value approximating the cerebral blood flow prediction value.

FIG. 9 is a diagram illustrating another aspect of training a second cerebral blood flow prediction model according to the other embodiment of the present disclosure.

Similarly to the description provided with reference to FIG. 8, the second cerebral blood flow prediction model may be trained on the basis of a second training dataset including first data (Node/connectivity info (x, y, z, c) of FIG. 9) related to 3D morphological data in the form of a 1D network model corresponding to a cerebrovascular region, second data (Boundary info (bci) of FIG. 9) related to boundary information of the cerebrovascular region, third data related to initial condition and boundary condition information (Initial/boundary values (bcv) of FIG. 9), and fourth data (Flow data (v, p) of FIG. 9) related to a blood flow speed and pressure.

Meanwhile, according to an aspect, the cerebral blood flow computation device 1000 may calculate a cerebral blood flow prediction value using a 1D network model and acquire the fourth data (Flow data (v, p) of FIG. 9) related to a blood flow speed and pressure. As an example, the cerebral blood flow computation device 1000 may compute a cerebral blood flow prediction value regarding a cerebral blood flow speed or pressure from the **1D** network model using any appropriate mathematical technique. For example, the cerebral blood flow computation device 1000 may be configured to assume cerebral blood flow to be Poiseuille flow and compute a cerebral blood flow prediction value regarding a cerebral blood flow speed or pressure using a governing equation.

Further, similarly to FIG. 8, the cerebral blood flow computation device 1000 may input the second training dataset that includes the first data composed of node information and connectivity information constituting the **1D** network model, the second data related to boundary information, the third data related to an initial condition and boundary condition, and the fourth data related to a cerebral blood flow prediction value, into the second cerebral blood flow prediction model and acquire an output value output from the second cerebral blood flow prediction model. Here, the cerebral blood flow computation device 1000 may compare the output value with the cerebral blood flow prediction value included in the fourth data and update parameters included in the second cerebral blood flow prediction model such that the second cerebral blood flow prediction model may output an output value approximating the cerebral blood flow prediction value.

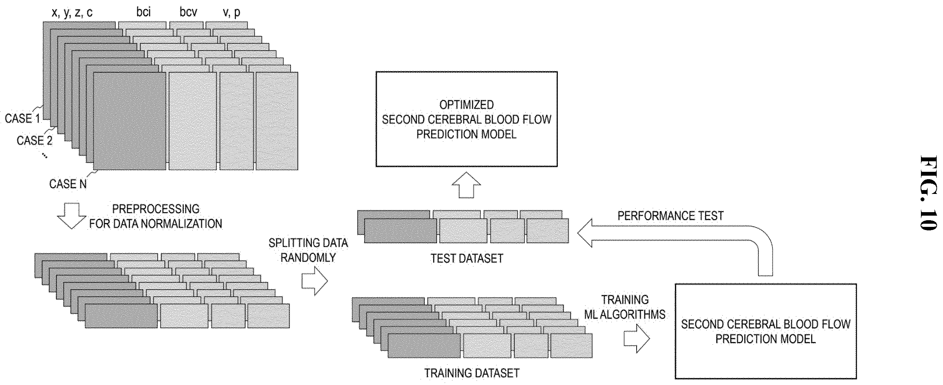

FIG. 10 is a diagram illustrating an aspect of training a second cerebral blood flow prediction model and an aspect of validating the performance of the second cerebral blood flow prediction model according to the other embodiment of the present disclosure.

The cerebral blood flow computation device 1000 according to the other embodiment of the present disclosure may perform an operation of preprocessing first data related to 3D morphological data (e.g., Node/connectivity info (x, y, z, c) of a region of interest), second data related to boundary information (bci) of the region of interest, third data related to an initial condition or boundary condition (bcv), and/or fourth data related to a cerebral blood flow prediction value (v, p), and the first to fourth data included in a second training dataset. For example, the cerebral blood flow computation device 1000 may normalize the first data, the second data, the third data, and/or the fourth data included in the second training dataset.

Further, the cerebral blood flow computation device 1000 according to the other embodiment of the present disclosure may perform an operation of splitting the preprocessed second training dataset into a training dataset and a test dataset or validation dataset. For example, the cerebral blood flow computation device 1000 may randomly split the preprocessed second training dataset into a training dataset and a test dataset. Here, the cerebral blood flow computation device 1000 may train the second cerebral blood flow prediction model through any ML algorithm using the training dataset. The cerebral blood flow computation device 1000 may test or validate the performance of the trained second cerebral blood flow prediction model using the validation dataset. For example, the cerebral blood flow computation device 1000 may compute the performance of the trained second cerebral blood flow prediction model, compare the computed performance with predetermined target performance, and train the second cerebral blood flow prediction model as described above until the computed performance becomes higher than the predetermined target performance, thereby acquiring an optimized second cerebral blood flow prediction model.

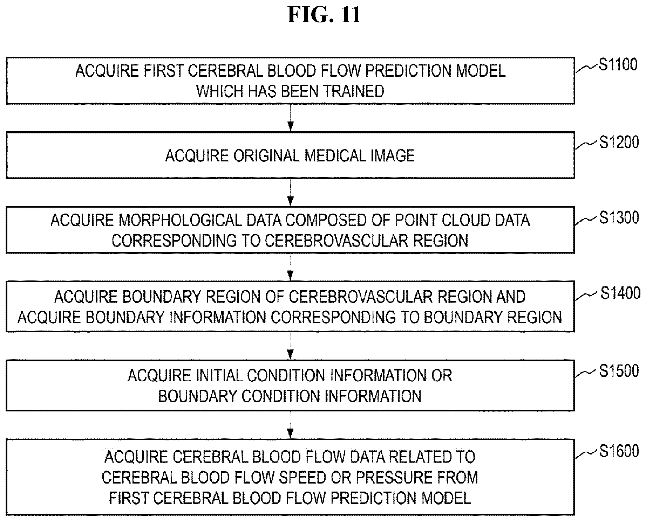

A method of computing cerebral blood flow data according to the embodiment of the present disclosure will be described in further detail below with reference to FIG. 11. In describing the method of computing cerebral blood flow data, details described with reference to FIGS. 3 to 6 may be omitted. However, this is merely for convenience of description and should not be construed as limiting due to omission.

The method of computing cerebral blood flow data according to the embodiment of the present disclosure may include an operation S 1100 of acquiring a first cerebral blood flow prediction model which has been trained, an operation S1200 of acquiring an original medical image, an operation S1300 of acquiring morphological data which corresponds to a cerebrovascular region and is composed of data in the form of a point cloud, an operation S1400 of acquiring a boundary region of the cerebrovascular region and acquiring boundary information corresponding to the boundary region, an operation S1500 of acquiring initial condition information or boundary condition information, and an operation S1600 of acquiring cerebral blood flow data related to a cerebral blood flow speed or pressure through the first cerebral blood flow prediction model.

In the operation S1100 of acquiring the first cerebral blood flow prediction model which has been trained, the cerebral blood flow computation device 1000 may acquire execution data for executing the first cerebral blood flow prediction model which has been trained. Here, the execution data may be any data for appropriately executing the first cerebral blood flow prediction model, including structural information, computational information, hierarchy information, and/or parameter information. Meanwhile, as described above, the cerebral blood flow computation device 1000 may be trained on the basis of a first training dataset including first data related to morphological data of a point cloud corresponding to a cerebrovascular region, second data related to boundary information of the cerebrovascular region, third data related to initial condition information and boundary condition information, and fourth data related to a cerebral blood flow prediction value regarding a blood flow speed or pressure. Specifically, the first cerebral blood flow prediction model may be configured to receive the first training dataset and output an output value from the first training dataset, and parameters included in the first cerebral blood flow prediction model may be updated to output the output value approximating the cerebral blood flow prediction value included in the fourth data such that the first cerebral blood flow prediction model may be trained.

According to an aspect, the fourth data may be generated by interpolating a blood flow speed or a blood flow pressure computed from the morphological data in the form of a point cloud corresponding to the cerebrovascular region to the morphological data having the form of a point cloud. Specifically, the blood flow speed or pressure may be computed using CFD on the basis of a surface model generated from the morphological data of a point cloud.

According to another aspect, the blood flow speed or pressure related to the fourth data may be computed from a 1D network model generated from the morphological data having the form of a point cloud using any mathematical technique (e.g., a governing equation).

In the operation S1200 of acquiring the original medical image, the cerebral blood flow computation device 1000 may acquire the original medical image through the transceiver unit 1100.

In the operation S1300 of acquiring the morphological data which corresponds to the cerebrovascular region and is composed of data in the form of a point cloud, the cerebral blood flow computation device 1000 may acquire a region of interest (e.g., a cerebrovascular region) using an image segmentation technique and acquire coordinate information corresponding to the region of interest. Further, the cerebral blood flow computation device 1000 may acquire 3D morphological data (i.e., a points model) composed of data in the form of a point cloud on the basis of the coordinate information of the region of interest.

In the operation S1400 of acquiring the boundary region of the cerebrovascular region and acquiring the boundary information corresponding to the boundary region, the cerebral blood flow computation device 1000 may acquire a boundary region of the region of interest (e.g., a region corresponding to cerebral blood vessels) and acquire boundary information (e.g., coordinate information of the boundary region) corresponding to the boundary region. Specifically, the cerebral blood flow computation device 1000 may acquire nodes corresponding to the boundary region of the region of interest (e.g., a cerebrovascular region) using an image segmentation technique and acquire the boundary information.

In the operation S1500 of acquiring the initial condition information or the boundary condition information, the cerebral blood flow computation device 1000 may acquire a 3D initial condition and/or boundary condition related to the boundary region.

In the operation S1600 of acquiring the cerebral blood flow data related to the cerebral blood flow speed or pressure through the first cerebral blood flow prediction model, the cerebral blood flow computation device 1000 may input the morphological data, the boundary information, and the initial condition information or the boundary condition information into the first cerebral blood flow prediction model and acquire cerebral blood flow data related to a cerebral blood flow speed or a cerebral blood flow pressure output from the first cerebral blood flow prediction model. Since the first cerebral blood flow prediction model is trained to output a cerebral blood flow prediction value related to a cerebral blood flow speed and pressure on the basis of points information, boundary information, and/or 3D initial condition and boundary condition information, the first cerebral blood flow prediction model which has been trained can output cerebral blood flow data related to a cerebral blood flow speed and pressure from 3D morphological data in the form of a point cloud corresponding to a cerebrovascular region, boundary information of the cerebrovascular region, and/or initial condition and/or boundary condition information.

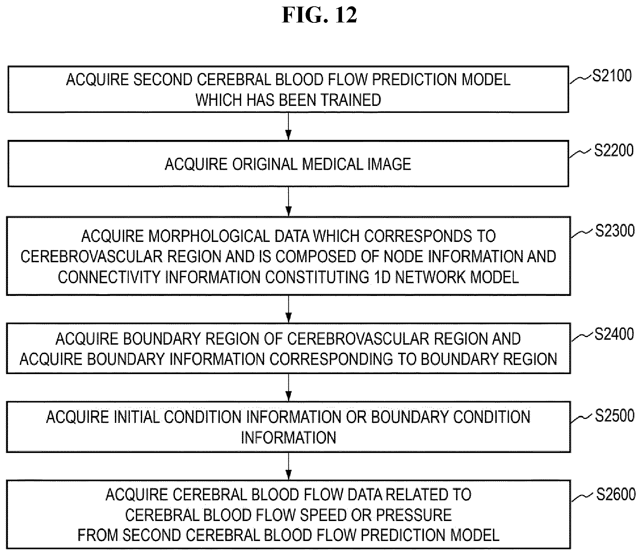

A method of computing cerebral blood flow data according to the other embodiment of the present disclosure will be described in further detail below with reference to FIG. 12. In describing the method of computing cerebral blood flow data, details described with reference to FIGS. 7 to 10 may be omitted. However, this is merely for convenience of description and should not be construed as limiting due to omission.

The method of computing cerebral blood flow data according to the other embodiment of the present disclosure may include an operation S2100 of acquiring a second cerebral blood flow prediction model which has been trained, an operation S2200 of acquiring an original medical image, an operation S2300 of acquiring morphological data which corresponds to a cerebrovascular region and is composed of node information and connectivity information constituting a 1D network model, an operation S2400 of acquiring a boundary region of the cerebrovascular region and acquiring boundary information corresponding to the boundary region, an operation S2500 of acquiring initial condition information or boundary condition information, and an operation S2600 of acquiring cerebral blood flow data related to a cerebral blood flow speed or pressure through the second cerebral blood flow prediction model.

In the operation S2100 of acquiring the second cerebral blood flow prediction model which has been trained, the cerebral blood flow computation device 1000 may acquire execution data for executing the trained second cerebral blood flow prediction model through the transceiver unit 1100. Here, the execution data may be any data required for appropriately executing the second cerebral blood flow prediction model, including computational information, hierarchy information, and/or parameter information. Meanwhile, as described above, the cerebral blood flow computation device 1000 may be trained on the basis of a second training dataset including first data related to node information and connectivity information constituting a 1D network model, second data related to boundary information of the cerebrovascular region, third data related to initial condition information or boundary condition information, and fourth data related to a cerebral blood flow prediction value regarding a blood flow speed or pressure. Specifically, the second cerebral blood flow prediction model may be configured to receive the second training dataset and output an output value from the second training dataset, and parameters included in the second cerebral blood flow prediction model may be updated to output the output value approximating the cerebral blood flow prediction value included in the fourth data such that the second cerebral blood flow prediction model may be trained.

According to an aspect, the fourth data may be generated by interpolating a blood flow speed or a blood flow pressure computed from morphological data in the form of a point cloud corresponding to the cerebrovascular region to morphological data having the form of a 1D network model. Specifically, the blood flow speed or pressure may be computed using CFD on the basis of a surface model generated from the morphological data of in the form of a point cloud.

According to another aspect, the blood flow speed or pressure related to the fourth data may be computed from morphological data in the form of a 1D network model composed of node information and connectivity information using any mathematical technique (e.g., a governing equation).

In the operation S2200 of acquiring the original medical image, the cerebral blood flow computation device 1000 may acquire the original medical image through the transceiver unit 1100.

In the operation S2300 of acquiring the morphological data which corresponds to the cerebrovascular region and is composed of the node information and connectivity information constituting a 1D network model, the cerebral blood flow computation device 1000 may acquire a region of interest (e.g., a cerebrovascular region) from the original medical image using an image segmentation technique and acquire coordinate information corresponding to the region of interest having the form of a point cloud. Here, the cerebral blood flow computation device 1000 may connect points included in the coordinate information corresponding to the cerebrovascular region to generate a 1D network model composed of node information corresponding to the region of interest and connectivity information presenting connection between nodes.

In the operation S2400 of acquiring the boundary region of the cerebrovascular region and acquiring the boundary information corresponding to the boundary region, the cerebral blood flow computation device 1000 may acquire boundary region of the region of interest (e.g., a region corresponding to cerebral blood vessels) and acquire boundary information (e.g., coordinate information of the boundary region) corresponding to the boundary region. Specifically, the cerebral blood flow computation device 1000 may acquire nodes corresponding to the boundary region of the region of interest (e.g., a cerebrovascular region) using an image segmentation technique and acquire the boundary information.

In the operation S2500 of acquiring the initial condition information or the boundary condition information, the cerebral blood flow computation device 1000 may acquire a 3D initial condition and/or boundary condition related to the boundary region.

In the operation S2600 of acquiring the cerebral blood flow data related to the cerebral blood flow speed or pressure through the second cerebral blood flow prediction model, the cerebral blood flow computation device 1000 may input the morphological data composed of node information and connectivity information, the boundary information, and the initial condition information or the boundary condition information into the second cerebral blood flow prediction model and acquire cerebral blood flow data related to a cerebral blood flow speed or a cerebral blood flow pressure output from the second cerebral blood flow prediction model. Since the second cerebral blood flow prediction model is trained to output a cerebral blood flow prediction value related to a cerebral blood flow speed and pressure on the basis of the node information and the connectivity information constituting the 1D network, the boundary information, and/or the 3D initial condition and boundary condition information, the second cerebral blood flow prediction model which has been trained can output cerebral blood flow data related to a cerebral blood flow speed and pressure from the morphological data of a cerebrovascular region composed of the node information and the connectivity information of the 1D network model, the boundary information of the cerebrovascular region, and/or the initial condition and/or boundary condition information.

With a method of computing cerebral blood flow data, a method of training a cerebral blood flow prediction model, and a cerebral blood flow data computation device according to an embodiment of the present disclosure, it is possible to train a cerebral blood flow prediction model capable of computing cerebral blood flow information from a point model or a 1D network model rather than a surface model or a solid model.

With a method of computing cerebral blood flow data, a method of training a cerebral blood flow prediction model, and a cerebral blood flow data computation device according to an embodiment of the present disclosure, it is possible to automatically compute cerebral blood flow information from a point model or a 1D network model rather than a surface model or a solid model using a cerebral blood flow prediction model which has been trained.

Further, with a method of computing cerebral blood flow data, a method of training a cerebral blood flow prediction model, and a cerebral blood flow data computation device according to an embodiment of the present disclosure,

the various operations of the cerebral blood flow computation device 1000 described above may be stored in the memory 1200 of the cerebral blood flow computation device 1000, and the processor 1300 of the cerebral blood flow computation device 1000 may perform the operations stored in the memory 1200.

The features, structures, effects, and the like described in the above embodiments are included in at least one embodiment of the present disclosure and are not necessarily limited to only one embodiment. Further, the features, structures, effects, and the like illustrated in each embodiment can be combined or modified for implementation in other embodiments by those of ordinary skill in the art to which the embodiments pertain. Accordingly, the contents related to such combinations and modifications should be construed as falling within the scope of the present disclosure.

Although embodiments have been mainly described above, these are merely illustrative and do not limit the present disclosure. Those skilled in the art to which the present disclosure pertains should understand that several modifications and applications that have not been described above can be made without departing from the fundamental characteristics of the present embodiments. In other words, each component specifically presented in the embodiments may be modified for implementation. In addition, differences associated with these modifications and applications are to be construed as falling within the scope of the present disclosure defined by the appended claims.

### Industrial Applicability

The above-described method of computing cerebral blood flow data, method of training a cerebral blood flow prediction model, and cerebral blood flow data computation device can be applied to the field of medical service for providing a medical service.

## Claims

1. A method of computing cerebral blood flow data by a cerebral blood flow computation device, the method comprising:
acquiring a cerebral blood flow prediction model which has been trained;
acquiring an original medical image;
acquiring morphological data, which is composed of data in the form of a point cloud, corresponding to a cerebrovascular region from the original medical image;
acquiring a boundary region of the cerebrovascular region and acquiring boundary information corresponding to the boundary region;
acquiring initial condition information or boundary condition information; and
inputting the morphological data, the boundary information, and the initial condition information or the boundary condition information into the cerebral blood flow prediction model and acquiring cerebral blood flow data related to a cerebral blood flow speed or a cerebral blood flow pressure output from the cerebral blood flow prediction model.

2. The method of claim 1, wherein the cerebral blood flow prediction model is trained on the basis of a training dataset that includes first data related to morphological data in the form of a point cloud corresponding to a cerebrovascular region, second data related to boundary information of the cerebrovascular region, third data related to initial condition information or boundary condition information, and fourth data related to a cerebral blood flow prediction value regarding a cerebral blood flow speed or pressure.

3. The method of claim 2, wherein the cerebral blood flow prediction model is configured to receive the training dataset and output an output value from the training dataset, and
parameters included in the cerebral blood flow prediction model are updated to output the output value approximating the cerebral blood flow prediction value such that the cerebral blood flow prediction model is trained.

4. The method of claim 3, wherein the fourth data is generated by interpolating a blood flow speed or a blood flow pressure computed from the morphological data corresponding to the cerebrovascular region in the morphological data having the form of a point cloud, and
the computed blood flow speed or blood flow pressure is computed on the basis of a surface model generated from the morphological data using computational fluid dynamics (CFD).

5. The method of claim 3, wherein the fourth data is generated by interpolating a blood flow speed or a blood flow pressure computed from the morphological data corresponding to the cerebrovascular region in the morphological data having the form of a point cloud, and
the computed blood flow speed or blood flow pressure is computed on the basis of a one-dimensional (1D) network model generated from the morphological data.

6. A method of computing cerebral blood flow data by a cerebral blood flow computation device, the method comprising:
acquiring a cerebral blood flow prediction model which has been trained;
acquiring an original medical image;
acquiring first morphological data, which is composed of node information and connectivity information constituting a one-dimensional (1D) network model, corresponding to a cerebrovascular region from the original medical image;
acquiring a boundary region of the cerebrovascular region and acquiring boundary information corresponding to the boundary region;
acquiring initial condition information or boundary condition information; and
inputting the first morphological data, the boundary information, and the initial condition information or the boundary condition information into the cerebral blood flow prediction model and acquiring cerebral blood flow data related to a cerebral blood flow speed or a cerebral blood flow pressure output from the cerebral blood flow prediction model.

7. The method of claim 6, wherein the cerebral blood flow prediction model is trained on the basis of a training dataset that includes first data composed of node information and connectivity information constituting a **1D** network model, second data related to boundary information of the cerebrovascular region, third data related to initial condition information or boundary condition information, and fourth data related to a cerebral blood flow prediction value regarding a cerebral blood flow speed or pressure.

8. The method of claim 7, wherein the cerebral blood flow prediction model is configured to receive the training dataset and output an output value from the training dataset, and
parameters included in the cerebral blood flow prediction model are updated to output the output value approximating the cerebral blood flow prediction value such that the cerebral blood flow prediction model is trained.

9. The method of claim 8, wherein the fourth data is generated by interpolating a blood flow speed or a blood flow pressure computed from second morphological data having the form of a point cloud corresponding to the cerebrovascular region in the first morphological data having the form of the **1D** network model, and
the computed blood flow speed or blood flow pressure is computed on the basis of a surface model generated from the second morphological data using computational fluid dynamics (CFD).

10. The method of claim 8, wherein the fourth data is generated by computing a cerebral blood flow prediction value related to a blood flow speed or a blood flow pressure from the first morphological data.

11. A computer-readable recording medium on which a program for causing a computer to execute the method of any one of claims 1 to 10 is recorded.
